# EUROPEAN PATENT SPECIFICATION

(11) **EP 0 425 487 B1**
(45) Date of publication and mention of the grant of the patent: **22.12.1993**
(21) Application number: 89902965.6
(22) Date of filing: 27.02.1989
(51) Int. Cl.: G01N 33/22

(54) **METHOD AND APPARATUS FOR DETERMINING THE ABILITY OF HEAVY FUELS TO SELF-IGNITE UNDER DIESEL CONDITIONS**
VORRICHTUNG UND VERFAHREN ZUR BESTIMMUNG DER NEIGUNG SCHWERER ÖLE ZUM SELBSTENTZÜNDEN UNTER DIESELBEDINGUNGEN
PROCEDE ET APPAREIL DETERMINANT LA CAPACITE D'AUTO-ALLUMAGE D'HUILES LOURDES DANS DES MOTEURS DIESEL

(30) Priority: 01.03.1988 NO 880890
(43) Date of publication of application: 08.05.1991
(73) Proprietor: SINVENT A/S, 0314 Oslo 3 (NO)
(72) Inventor: RUZICKA, Dalibor, J., N-1315 Nesöya (NO); OSTVOLD, Geir, N-5154 Mongstad (NO)
(74) Representative: Allard, Susan Joyce
(86) International application number: NO8900017
(87) International publication number: WO8908253

(56) References cited:
- EP-A- 0 143 571
- DE-C- 2 524 703
- US-A- 3 738 808
- US-A- 4 057 393

## Description

The present invention comprises a method and an apparatus for determining (as Cetane Number) the ability of heavy fuels to self-ignite under diesel conditions by determining the Cetane Number. Diesel conditions imply that the fuel is injected as small droplets into compressed hot air.

While the Octane Number for a gasoline/air mixture indicates the gasoline's ability to be ignited in a controlled manner, a fuel's Cetane Number indicates its ability to be ignited under diesel conditions,
Cetane Number has not been defined for heavy fuels. Strictly it is therefore not correct to use this designation for the Ignition Number determined for such fuels, irrespective of the method employed. However, the Ignition Number determined for heavy fuels expresses the same property of heavy fuels as the Cetane Number does for distillates, since the scale for Ignition Number has been established by calibration with distillates of known Cetane Number. For the sake of simplicity, the term Cetane Number will be used also for heavy fuels in the description below.

Users of large marine engines have for a long time felt the need for a fast and reliable method for the determination of Cetane Number, since this gives a good indication of a fuel's autoignitibility. When fuels of varying quality are used, the knowledge of Cetane Number may be of primary importance, since the engine can be adjusted according to the properties of the fuel. It would be of great advantage for the Chief Engineer if the test method was so fast that the measurement might be carried out at the beginning of the bunkering procedure and the necessary apparatus so low priced that it might be kept aboard at all times. In this manner it would be possible for the Chief Engineer to determine the quality of the fuel at a sufficiently early stage for the bunkering to be interrupted if the fuel should appear to be of unacceptably low quality. The danger of a total engine breakdown would thus be almost completely eliminated.

Hitherto an estimation of ignition quality has not been possible, since the available methods are too time-consuming and require skilled laboratory personnel.

The standard method of determination of Cetane Number is an engine test where comparison with fuels of known Cetane Number is carried out in a special engine (CFR). Such a method is obviously not suitable for routine measurements, for example during bunkering.

Simpler and less costly tests have been used in laboratories recently. These methods involve heating up a fuel sample in a controlled atmosphere and measuring one or more parameters as a function of temperature.

For example, tests have been carried out in which the weight change was recorded while the sample was heated (thermogravimetry), but it has not been possible to give a satisfactory interpretation of the results. In addition the method requires expensive and delicate instrumentation and a skilled operator.

Attempts have also been made to combine thermogravimetry and differential thermal analysis or differential scanning calorimetry, where temperature changes and energy changes are measured, respectively. It was possible to interpret such measurements in terms of Ignition Number (Liddy et al.), however the apparatus is very costly and delicate and an expert is required to carry out and interpret the analysis.

Attempts have also been made to apply a modified DIN method for the characterization of heavy fuels. However, the equipment is not simple to use and has not been developed into a commercial product.

In another attempt simulation of a diesel cylinder by a combustion bomb has been used to determine the ignition quality of fuel oils. A model which probably will be suitable for laboratory use, will apparently be marketed. It is, however, expected that the equipment will be too large and complicated for use aboard ships and, to our present knowledge, no reliable correlation has been established between the ignition delay determined in the bomb and that in a real engine.

From the above it is apparent that all available methods for the determination of igniton properies of heavy fuels have weaknesses which make them unsuitable for use aboard ships. The methods are both costly and complicated, in many cases involve the use of delicate equipment and in most cases require expert operators. No good correlation been demonstrated between the measured and observed data for some of the methods.

The present invention eliminates all the above mentioned shortcomings, since it offers a method which can be carried out aboard during bunkering and an apparatus which can be utilized by non-experts, the apparatus is neither costly nor demands any large space, so that it can be permanently installed aboard any ship. It is light and robust, it can be used under the influence of acceleration forces and it can be completely automatic. Above all, the method and the apparatus according to the invention, yield data which correlate very well with those measured directly in engines.

More precisely, the invention comprises a method for the determination of autoignitibility of heavy fuels under diesel conditions by the determination of Cetane Number, the procedure being the following. In a first stage, a known amount of an oil sample is partially oxidized in an oxidizing gas while gradually increasing the temperature. In a second stage, the partially oxidized oil components are oxidized catalytically to CO2 and water while a detector continuously monitors the produced amount of CO2 as a function of temperature, after which a computer algoritm based on statistical multivariate principal component analysis, yields the Cetane Number directly from the recorded amounts of CO2.

The invention also comprises an apparatus in which the above procedure can be carried out, the characteristics of the apparatus being the following:
- a main reactor in which a small crucible for the oil sample can be placed, the reactor also including means for the introduction of the oxidizing gas and means for the emission of the oxidizing gas and oxidation products,
- a second reaction chamber which comprises a catalyst for the second stage oxidation of the products of partial oxidation to CO2 and water and means for the emission of the reaction gases,
- a chamber including a detector for the detection of the CO2 content in the reaction gases,
- a processor unit which is able to generate the Cetane Number of the oil from the compiled CO2-values, using an algorithm based on multivariate principal component analysis, and also
- means for the heating of the oxidizing gas according to a preset temperature programme.

The oxidizing gas can be any gas which is able to bring about the desired oxidation of the oil. It is thus possible to utilize pure oxygen, air or any mixture of oxygen with gases which are inert with respect to the oil.

The temperature interval for oxidation and the rate of temperature increase of the oil sample are chosen in such a manner that the whole temperature region in which oxidation of the various components can take place is included, so that the generated data are reproducible and yield a good correlation with engine data. At the same time it is desirable that the analysis time be as short as possible.

Reproducible data are obtained when the oxidation is carried out in the temperature region 35 to 750°C in the first stage.

It has also been verified that heating rates of 20 to 50 °C/min give very good results. These values are thus preferred for carrying out the analysis, and they imply an analysis time in the region of 15 - 35 minutes.

A good agreement between the Cetane Number determined by the analysis and that determined by engine test has been obtained using a heating rate as high as 40°C/min, which corresponds to an analysis time of ca 18 minutes. These parameters thus represent preferred conditions for the method according to the invention.

The second stage catalytic oxidation is not very critical with respect to the choice of temperature, but the latter must be sufficiently high to convert all oxidizable components to CO2 and water in this stage. From the practical point of view the necessary temperature depends - among other factors - on the type of catalyst used and on the geometry of the second stage reactor. In laboratory experiments a platinum catalyst used at ca 1000°C was found to be satisfactory.

The choice of catalyst is not critical for the method according to the invention, so that any available commercial catalyst for the oxidation of hydrocarbons can be utilized. Catalysts with platinum as the active component were found to perform well. For the sake of economy and to obtain a high surface area/mass ratio for the catalyst, it is preferable that the active component is present in small quantities on the surface of a porous ceramic support.

It is also possible to add a cracking catalyst to the oil sample to be analysed, by which means it is possible to lower the final temperature from ca 750 to ca 620°C. At the same time the amount of residue which remains to be oxidized at the highest temperature, is smaller. Whether this represents a real time saving may depend on the ease of measuring the amount of the catalyst and the manner in which it is added.

The detector for the monitoring of CO2 may be an IR detector or another type of detector, for example one based on the the measurement of heat conductivity. Very good results have been obtained by the use of an IR detector and this is to be considered as the preferred type for the invention.

Fig. 1 shows a sketch drawing of the apparatus according to the invention.

Fig. 2 shows a correlation between Cetane Numbers determined by engine tests and by analysis for a number of oil samples. The experimental conditions are specified in the examples.

A more detailed description of the method and the apparatus is given below, with reference to figures.

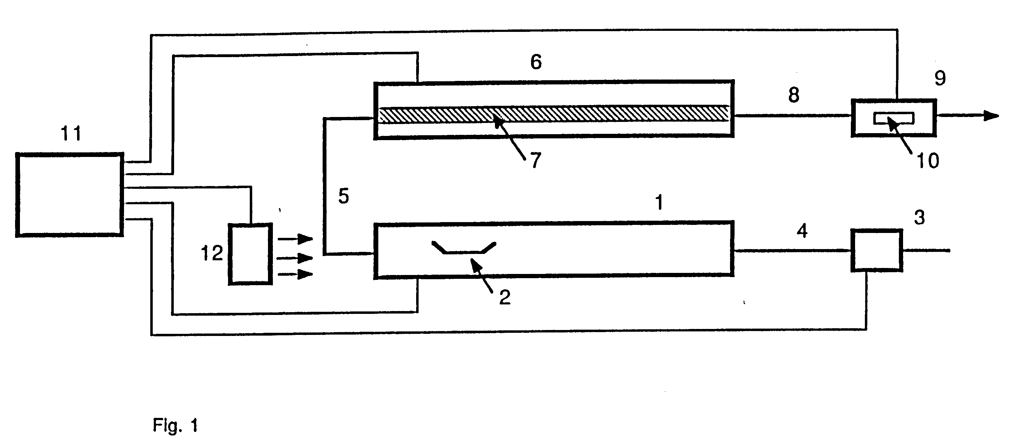

Fig. 1 shows the main reactor (1), which contains a crucible (2) for the oil sample, the oxidizing gas is supplied by a pump (3) through a tubing (4). The oxidizing gas and the oxidation products pass from the main reactor (1) to the second reaction chamber (6) through a rigid tube (5). The oxidation products are completely converted to CO2 and water in the second reaction chamber (6) by means of a catalyst (7). The gases then pass into a chamber (9) containing a detector (10) for quantitative determination of CO2. A processor unit (11) connected to the detector yields the Cetane Number directly from the recorded amounts of CO2. The processor unit can also be connected to the pump (3) to control the air flow, as well as the power to facilitate the temperature rise in the main reactor (1) and the temperature in the second reaction chamber (6).

The processor unit (11) can also control other process variables. It is, for example, an advantage to utilize a cooling fan (12) in conjunction with the main reactor (1), to be able to cool down the latter quickly between two subsequent analyses. The processor unit can control the start and stop of the cooling period.

Since the main reactor (1) is operated at at relatively low temperature during a certain part of the analysis period, and the second reactor (6) is always at a considerably higher temperature, a thermal barrier (not shown) is used between the two reactors.

The invention is illustrated below by an example which shows the correlation between Cetane Numbers detemined by analysis according to the invention, and Cetane Numbers determined by engine tests for the same oils.

### Example:

21 different oil samples for which Ignition Numbers (Cetane Numbers) were determined by an engine test, were analysed by the method and apparatus according to the invention. The analysis parameters were kept constant and were the following:

| | |
|---|---|
| oxidation gas: air: | 100 ml/min |
| amount of oil | 25 mg |
| initial temperature | 35°C |
| final temperature | 750°C |
| heating rate | 40°C/min |
| time for analysis | 18 min |

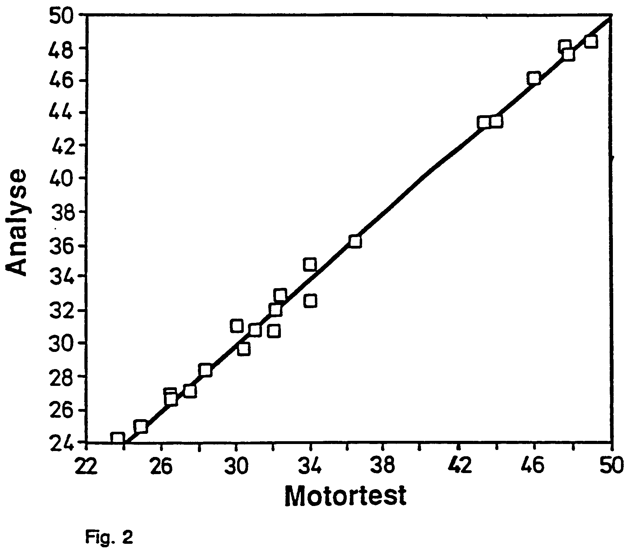

A statistical model based on multivariate principal component analysis has been developed from the analytical data. The model was then optimized to yield an unambiguous correlation between the Cetane Numbers from engine tests and analyses, respectively. The correlation is shown in Fig. 2.

## Claims

1. A method for the determination of the autoignitibility of heavy fuels under diesel conditions by determining the Cetane Number, **characterized in that** in a first stage, a known amount of an oil sample is partially oxidized in an oxidizing gas while gradually increasing the temperature; in a second stage, the partially oxidized oil components are oxidized catalytically to CO2 and water while a detector continuously monitors the produced amount of CO2 as a function of temperature, after which a computer algorithm based on statistical multivariate principal component analysis, yields the Cetane Number directly from the recorded amounts of CO2.

2. A method as claimed in claim 1, **characterized in that** in said first stage an initial temperature of 35 °C and a final temperature of 750 °C is employed.

3. A method as claimed in claim 1 or 2, **characterized in that** the temperature of said oxidizing gas in said first stage is increased at a rate of 20 - 50 °C/min during a period of 15 - 35 minutes.

4. A method as claimed in claim 1 or 2, **characterized in that** the temperature of said oxidizing gas in said first stage is increased at a rate of 40 °C/min during a period of about 18 minutes.

5. A method as claimed in claim 1, **characterized in that** said second stage catalytical oxidation is performed at 1000 °C.

6. A method as claimed in claim 1, **characterized in that** said catalyst used for the second stage oxidation is an oxidation catalyst.

7. A method as claimed in claim 6, **characterized in that** said catalyst contains platinum either alone, or as an active component on the surface of a ceramic support.

8. A method as claimed in claim 1, **characterized in that** said detector used for monitoring the CO2-content is an IR-detector.

9. Apparatus for the performance of the method as claimed in claim 1, **characterized** by the following:
a) a main reactor (1) in which a small container (2) for the oil sample can be placed, means (3,4) for the introduction of the oxidizing gas and means (5) for the emission of the oxidizing gas and oxidation products,
b) a second reaction chamber (6) which comprises a catalyst (7) for a second stage oxidation of the products of partial oxidation to CO2 and water, and means (8) for the emission of the reaction gases,
c) a chamber (9) including a detector (10) for the monitoring of the CO2 content in the reaction gases,
d) a processor unit (11) programmed to generate the Cetane Number of the oil from the recorded CO2-values, using an algorithm based on mulitvariate principal component analysis, and also
e) means for the heating of the oxidizing gas according to a preset temperature programme.

10. Apparatus as claimed in claim 9, **characterized in that** said detector for the monitoring of the CO2-content is an IR-detector.

## Patentansprüche

1. Verfahren zum Bestimmen der Selbstentzündlichkeit schwerflüchtiger Brennstoffe unter Dieselbedingungen durch Bestimmen der Cetanzahl,
dadurch **gekennzeichnet,** daß einem ersten Schritt eine bekannte Menge einer Ölprobe in einem oxidierenden Gas partiell oxidiert wird, während die Temperatur allmählich erhöht wird; daß in einem zweiten Schritt die partiell oxidierten Ölbestandteile katalytisch zu CO₂ und Wasser oxidiert werden, während ein Detektor fortlaufend die erzeugte Menge von CO₂ als Funktion der Temperatur überwacht, wonach ein auf einer statistischen, multivariablen Hauptkomponentenanalyse beruhender Computeralgorithmus die Cetanzahl direkt ausgehend von den registrierten CO₂-Mengen liefert.

2. Verfahren nach Anspruch 1,
dadurch **gekennzeichnet,** daß in dem ersten Schritt eine Anfangstemperatur von 35°C und eine Endtemperatur von 750°C verwendet wird.

3. Verfahren nach Anspruch 1 oder 2,
dadurch **gekennzeichnet,** daß die Temperatur des oxidierenden Gases in dem ersten Schritt mit einer Rate von 20 bis 50°C/min während eines Zeitraums von 15 bis 35 min erhöht wird.

4. Verfahren nach Anspruch 1 oder 2,
dadurch **gekennzeichnet,** daß die Temperatur des oxidierenden Gases in dem ersten Schritt mit einer Rate von 40°C/min während eines Zeitraums von etwa 18 min erhöht wird.

5. Verfahren nach Anspruch 1,
dadurch **gekennzeichnet,** daß die katalytische Oxidation in dem zweiten Schritt bei 1000°C durchgeführt wird.

6. Verfahren nach Anspruch 1,
dadurch **gekennzeichnet,** daß der für die Oxidation in dem zweiten Schritt verwendete Katalysator ein Oxidationskatalysator ist.

7. Verfahren nach Anspruch 6,
dadurch **gekennzeichnet,** daß der Katalysator entweder lediglich Platin oder Platin als aktiven Bestandteil auf der Oberfläche eines keramischen Trägers enthält.

8. Verfahren nach Anspruch 1,
dadurch **gekennzeichnet,** daß der zum Überwachen des CO₂-Gehalts verwendete Detektor ein IR-Detektor ist.

9. Gerät zum Durchführen des Verfahrens nach Anspruch 1,
**gekennzeichnet** durch folgende Merkmale:
a) einen Hauptreaktor (1), in den ein kleiner Behälter (2) für die Ölprobe gestellt werden kann, mit Einrichtungen (3), (4) zum Einführen des oxidierenden Gases und Einrichtungen (5) zum Anführen des oxidierenden Gases und der Oxidationsprodukte,
b) eine zweite Reaktionskammer (6), die einen Katalysator (7) zur Oxidation der Produkte der partiellen Oxidation in einem zweiten Schritt zu CO₂ und Wasser sowie Einrichtungen (8) zum Anführen der Reaktionsgase aufweist,
c) eine Kammer (9) mit einem Detektor (10) zum Überwachen des CO₂-Gehalts der Reaktionsgase,
d) eine Prozessoreinheit (11), die so programmiert ist, daß sie ausgehend von den registrierten CO₂-Werten, die Cetanzahl des Öls liefert, wobei sie einen auf der multivariablen Hauptkomponentenanalyse beruhenden Algorithmus verwendet,
e) Einrichtungen zum Heizen des oxidierenden Gases entsprechend einem voreingestellten Temperaturprogramms.

10. Gerät nach Anspruch 9,
dadurch **gekennzeichnet,** daß der Detektor zum Überwachen des CO₂-Gehalts ein IR-Detektor ist.

## Revendications

1. Procédé de détermination de l'auto-inflammabilité d'huiles lourdes dans des conditions Diesel par détermination de l'Indice de Cétane, caractérisé en ce que, au cours d'un premier stade, une quantité connue d'un échantillon d'huile est partiellement oxydée dans un gaz oxydant, la température étant progressivement augmentée; au cours d'un second stade, les composants de l'huile partiellement oxydée sont oxydés catalytiquement en CO₂ et eau, un détecteur surveillant continûment la quantité de CO₂ produite en fonction de la température, après quoi un algorithme informatisé reposant sur une analyse statistique à plusieurs variables du principal composant donne directement l'Indice de Cétane d'après les quantités de CO₂ relevées.

2. Procédé selon la revendication 1, caractérisé en ce que, au cours dudit premier stade, on utilise une température initiale de 35°C et une température finale de 750°C.

3. Procédé selon la revendication 1 ou 2, caractérisé en ce que la température dudit gaz oxydant au cours dudit premier stade est augmentée à raison de 20 à 50°C/min pendant une durée de 15 à 35 min.

4. Procédé selon la revendication 1 ou 2, caractérisé en ce que la température dudit gaz oxydant au cours dudit premier stade est augmentée à raison de 40°C/min pendant une durée d'environ 18 min.

5. Procédé selon la revendication 1, caractérisé en ce que ladite oxydation catalytique du second stade est effectuée à 1 000°C.

6. Procédé selon la revendication 1, caractérisé en ce que ledit catalyseur utilisé pour l'oxydation du second stade est un catalyseur d'oxydation.

7. Procédé selon la revendication 6, caractérisé en ce que ledit catalyseur contient du platine, soit seul, soit sous forme de composant actif à la surface d'un support céramique.

8. Procédé selon la revendication 1, caractérisé en ce que ledit détecteur utilisé pour surveiller la teneur en CO₂ est un détecteur IR.

9. Appareil pour la mise en oeuvre du procédé selon la revendication 1, caractérisé par les éléments suivants :
a) un réacteur principal (1) dans lequel on peut placer un petit récipient (2) destiné à l'huile, des moyens (3, 4) destinés à l'introduction du gaz oxydant et un moyen (5) destiné à l'émission du gaz oxydant et des produits d'oxydation
b) une seconde chambre de réaction (6) comprenant un catalyseur (7) destiné à l'oxydation du second stade des produits de l'oxydation partielle en CO₂ et eau et un moyen (8) destiné à l'émission des gaz réactionnels
c) une chambre (9) comprenant un détecteur (10) destiné à la surveillance de la teneur en CO₂ des gaz réactionnels
d) une unité de traitement (11) programmée pour calculer l'Indice de Cétane de l'huile d'après les valeurs relevées pour CO₂, à l'aide d'un algorithme reposant sur une analyse à plusieurs variables du principal composant et également
e) un moyen destiné à chauffer le gaz oxydant selon un programme de températures présélectionné.

10. Appareil selon la revendication 9, caractérisé en ce que ledit détecteur destiné à surveiller la teneur en CO₂ est un détecteur IR.
